(19) Europäisches Patentamt
European Patent Office
Office européen des brevets

(11) **EP 4 529 826 A1**

(12) **EUROPEAN PATENT APPLICATION**

(43) Date of publication:
02.04.2025 Bulletin 2025/14

(21) Application number: 24201441.3

(22) Date of filing: 19.09.2024

(51) International Patent Classification (IPC):
*A61B 1/06* (2006.01)      *A61B 1/07* (2006.01)

(52) Cooperative Patent Classification (CPC):
A61B 1/0669; A61B 1/063; A61B 1/0638;
A61B 1/07

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 US 202318373536

(71) Applicant: Olympus Corporation
Hachioji-shi
Tokyo 192-8507 (JP)

(72) Inventors:
• HANANO, Kazunari
Tokyo, 192-8507 (JP)

• MURAYAMA, Kazuaki
Tokyo, 192-8507 (JP)
• USHIO, Yasuaki
Tokyo, 192-8507 (JP)
• ANDERS, Konrad
22045 Hamburg (DE)
• BOCK, Mario
22045 Hamburg (DE)
• KLINDER, Jens
22045 Hamburg (DE)
• SCHOUWINK, Peter
22045 Hamburg (DE)

(74) Representative: Gunzelmann, Rainer
Wuesthoff & Wuesthoff
Patentanwälte und Rechtsanwalt PartG mbB
Schweigerstraße 2
81541 München (DE)

(54) **LIGHT SOURCE DEVICE FOR ENDOSCOPE**

(57)     A light source device for an endoscope includes a plurality of excitation laser light sources, a plurality of first condensing optical systems, a plurality of first optical fiber bundles on which the excitation light condensed by the plurality of first condensing optical systems is incident, an illumination light source that generates white light, a second condensing optical system that condenses the white light emitted from the illumination light source, a second optical fiber bundle, and a first glass rod of which a first end surface is connected to emission end surfaces of the first and second optical fiber bundles and first emission ends of the first optical fiber bundles and a second emission end of the second optical fiber bundle are bundled. A connecting portion of an illumination optical system of an endoscope is provided on a second end surface of the first glass rod opposite to the first end surface.

FIG. 2

EP 4 529 826 A1

**Description**

BACKGROUND OF THE INVENTION

Field of the Invention

The present invention relates to a light source device for an endoscope.

Description of Related Art

Examples of a light source device for an endoscope in the related art include a projection optical system disclosed in Japanese Unexamined Patent Application, First Publication No. 2016-191888. The projection optical system disclosed in Japanese Unexamined Patent Application, First Publication No. 2016-191888 includes a laser light source, a light guide, a rod, and a micro-prism array. A laser beam emitted from the laser light source is incident on a fiber bundle, is incident on the rod via a cover glass, and is incident on the micro-prism array. An object surface is irradiated with light emitted from the micro-prism array.

SUMMARY OF THE INVENTION

The invention is defined by the appended claims. A light source device for an endoscope according to an aspect of the present disclosure includes a plurality of excitation laser light sources, a first condensing optical system that is formed of a plurality of optical systems condensing excitation light generated from the plurality of excitation laser light sources, a plurality of first optical fiber bundles on which the excitation light condensed by the plurality of optical systems is incident, an illumination light source that generates white light, a second condensing optical system that condenses the white light emitted from the illumination light source, a second optical fiber bundle on which the light emitted from the illumination light source and condensed by the second condensing optical system is incident, and a first glass rod of which a first end surface is connected to emission end surfaces of the first and second optical fiber bundles. First emission ends of the first optical fiber bundles and a second emission end of the second optical fiber bundle are bundled. A connecting portion of an illumination optical system of an endoscope is provided on a second end surface of the first glass rod opposite to the first end surface.

BRIEF DESCRIPTION OF THE DRAWINGS

FIG. 1 is an overall view showing an endoscope that includes a light source device for an endoscope according to an embodiment.
FIG. 2 is a diagram showing the light source device for an endoscope according to the embodiment.
FIG. 3A is a diagram showing the relationship between an outer diameter of a glass rod and an outer diameter of a fiber.
FIG. 3B is a diagram showing the relationship between the outer diameter of the glass rod and the outer diameter of the fiber.
FIG. 4A is a diagram showing the relationship between an outer diameter of a glass rod and an outer diameter of a fiber.
FIG. 4B is a diagram showing the relationship between the outer diameter of a glass rod and the outer diameter of a fiber.
FIG. 5 is a diagram showing the relationship between an etendue and a diameter of a fiber.
FIG. 6 is a diagram showing the relationship between an etendue and a diameter of a fiber.
FIG. 7 is a diagram showing the relationship between an etendue and a diameter of a fiber.
FIG. 8 is a diagram showing the relationship between an etendue and a diameter of a fiber.
FIG. 9 is an overall view showing a modification example of the light source device for an endoscope according to the embodiment.

DETAILED DESCRIPTION OF THE INVENTION

(Embodiment)

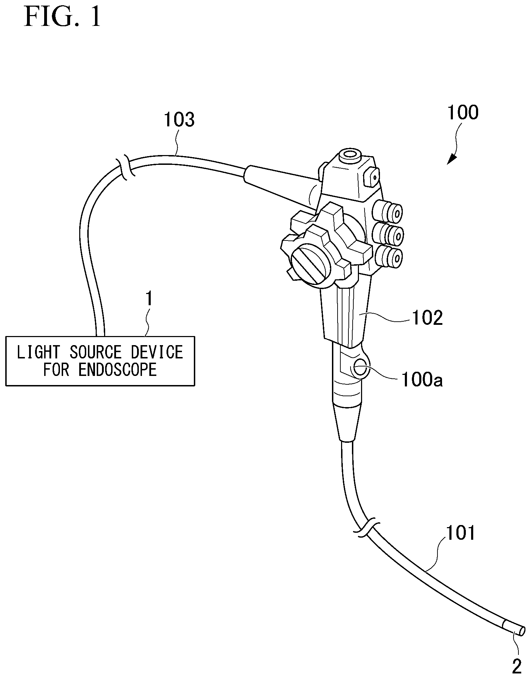

A light source device for an endoscope according to an embodiment of the present disclosure will be described with reference to FIGS. 1 to 8. As shown in FIG. 1, the light source device 1 for an endoscope is used for an endoscope 100.

[Endoscope]

The endoscope 100 is, for example, a device that is used to observe and treat the inside of the body of a patient lying on an operating table. As shown in FIG. 1, the endoscope 100 includes an elongated insertion part 101 that is to be inserted into the body of a patient, an operation part 102 that is connected to a proximal end of the insertion part 101, and a universal cord 103 that extends from the operation part 102.

A treatment tool insertion opening 100a is formed on a proximal end side of the insertion part 101. The treatment tool insertion opening 100a is connected to a proximal end portion of a treatment tool insertion channel (not shown) in the insertion part 101.

The treatment tool insertion opening 100a is an insertion opening through which a treatment tool (not shown) for an endoscope is to be inserted into the treatment tool insertion channel.

The operation part 102 receives an operation for the endoscope 100. The universal cord 103 connects the endoscope 100 and the light source device 1 for an endoscope according to the present embodiment.

A distal end of the insertion part 101 of the endoscope 100 is provided with an illumination optical system (not shown) on which light emitted from the light source device 1 for an endoscope is incident and an imaging unit 104.

[Light source device for endoscope]

The light source device 1 for an endoscope includes an excitation laser light source 10, a first condensing optical system 11, a first optical fiber bundle 12, an illumination light source 20, a second condensing optical system 21, a second optical fiber bundle 22, and a glass rod (first glass rod) 30.

The excitation laser light source 10 excites a photoreactive reagent. The excitation laser light source 10 includes a first LD 10A, a second LD 10B, and a third LD 10C.

The first LD 10A has an intensity in a blue light region and excites a predetermined biological material included in biological tissue. Specifically, the first LD 10A is adapted to generate light having an intensity in a wavelength range of, for example, 380 nm to 488 nm.

The second LD 10B is adapted to generate light having an intensity in a wavelength range of, for example, 488 nm to 680 nm.

The third LD 10C is adapted to generate red light having an intensity in a red light region. Specifically, the third LD 10C is adapted to generate light having an intensity in a wavelength range of, for example, 680 nm to 780 nm.

The wavelength range of the light emitted from the excitation laser light source 10 is merely an example.

The first condensing optical system 11 includes a first condenser lens (optical system) 11A that condenses excitation light generated from the first LD 10A, a second condenser lens (optical system) 11B that condenses excitation light generated from the second LD 10B, and a third condenser lens (optical system) 11C that condenses excitation light generated from the third LD 10C.

The first optical fiber bundle 12 includes on which the excitation light condensed by the first condenser lens 11A is incident, a second fiber 12B on which the excitation light condensed by the second condenser lens 11B is incident, and a third fiber 12C on which the excitation light condensed by the third condenser lens 11C is incident.

The illumination light source 20 is a white light source that emits white light. For example, a white LED, a white LD, a halogen lamp, a xenon lamp, or the like is used as the white light source. In the present embodiment, a white LED is used as the illumination light source 20.

The second condensing optical system 21 is formed of a condenser lens that condenses white light emitted from the illumination light source 20.

White light, which is emitted from the illumination light source 20 and is condensed by the second condensing optical system 21, is incident on the second optical fiber bundle 22.

In the present embodiment, each of the first fiber 12A, the second fiber 12B, the third fiber 12C, and the second optical fiber bundle 22 is a multi-core optical fiber.

Each of the first fiber 12A, the second fiber 12B, the third fiber 12C, and the second optical fiber bundle 22 may be formed of a single-core optical fiber.

Further, an outer peripheral surface of each of the first fiber 12A, the second fiber 12B, the third fiber 12C, and the second optical fiber bundle 22 is coated with a sheath to be kept watertight.

In the present embodiment, a laser is used as the excitation laser light source 10 and an LED is used as the illumination light source 20. For this reason, a spot diameter of light emitted from the illumination light source 20 is larger than a spot diameter of light emitted from the excitation laser light source 10. In order to make the light emitted from the illumination light source 20 be incident on the fiber without waste, an outer diameter of the second optical fiber bundle 22 is adapted to be larger than an outer diameter of the first optical fiber bundle 12.

Further, the first condensing optical system 11 and the second condensing optical system 21 are magnification optical systems in the present embodiment. At least the second condensing optical system 21 of the first condensing optical system 11 and the second condensing optical system 21 may be a magnification optical system.

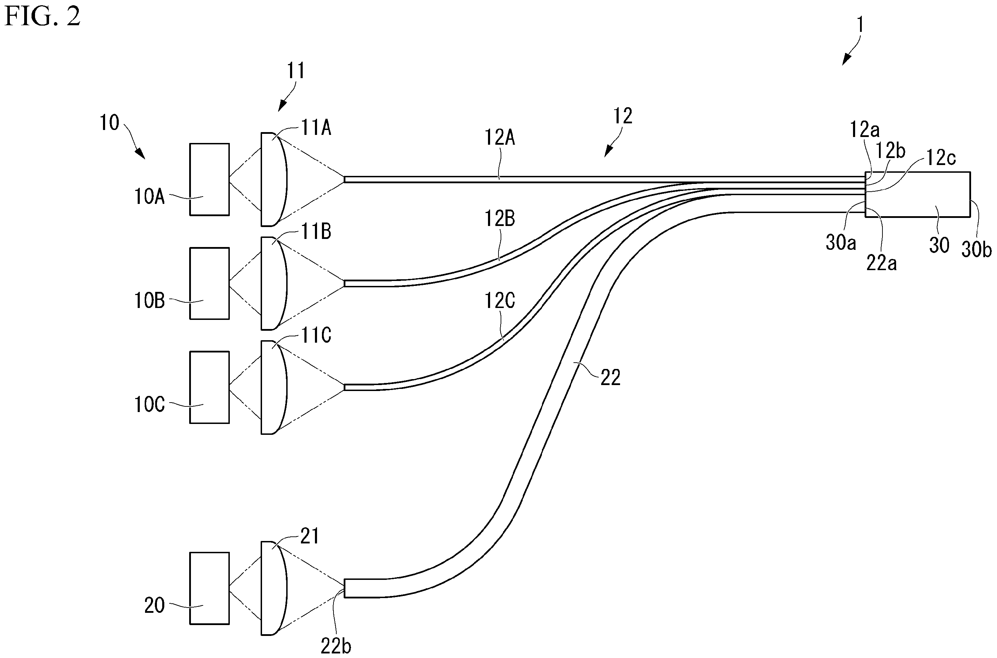

As shown in FIG. 2, the glass rod (first glass rod) 30 is disposed on an emission side of the first optical fiber bundle 12 and the second optical fiber bundle 22.

The first optical fiber bundle 12 and the second optical fiber bundle 22 are disposed such that an emission end surface 12a of the first fiber 12A, an emission end surface 12b of the second fiber 12B, an emission end surface 12c of the third fiber 12C, and an emission end surface 22a of the second optical fiber bundle 22 are flush with each other.

An incident end surface (first end surface) 30a of the glass rod is provided in contact with the respective emission end surfaces 12a to 12c of the first optical fiber bundle 12 and the emission end surface 22a of the second optical fiber bundle 22. Since the optical fiber bundles 12 and 22 are in contact with the glass rod 30, light emitted from the first optical fiber bundle 12 and the second optical fiber bundle 22 can be efficiently guided to the glass rod 30.

The glass rod 30 may be disposed on an emission end side of the first optical fiber bundle 12 and on an emission end side of the second optical fiber bundle 22 to have a gap between an incident end surface 30a of the glass rod 30 and the respective emission end surfaces 12a to 12c of the first optical fiber bundle 12 and the emission end surface 22a of the second optical fiber bundle 22.

An emission end surface (second end surface) 30b of the glass rod 30 is connected to a connecting portion (not shown) of an illumination optical system provided in the universal cord 103 of the endoscope 100.

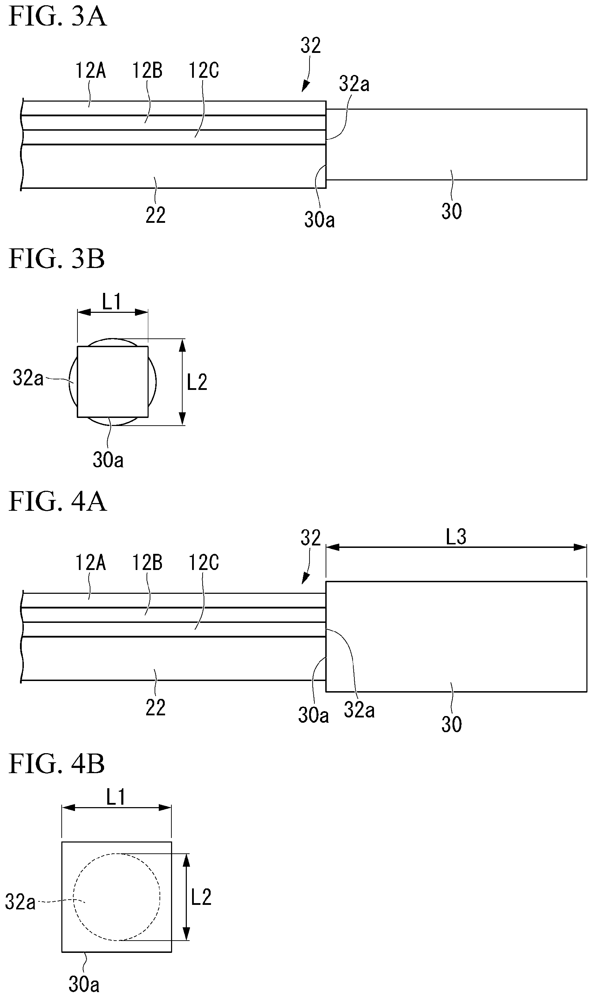

The shape of the glass rod is the shape of a square prism in the present embodiment. As shown in FIGS. 4A and 4B, the shape of a cross-section of the glass rod orthogonal to a longitudinal direction of the glass rod is a square shape. Further, the emission ends (hereinafter, referred to an emission end of a fiber 32) of the first optical fiber bundle 12 and the second optical fiber bundle 22 are bundled in a circular shape.

Next, the relationship between the length of one side of the glass rod and the emission end surface of an emission end of the fiber will be described with reference to FIGS. 3A to 4B.

The length of one side of the glass rod is denoted by L1, and the diameter of an emission end surface 32a of the fiber 32 is denoted by L2. The relationship between the length L1 of one side of the glass rod and the diameter L2 of the emission end surface 32a is such that the length L1 of one side of the glass rod 30 is within a range larger or smaller than the diameter L2 of the emission end surface 32a by 10%.

First, a case where the length L1 of one side of the glass rod 30 is smaller than the diameter L2 of the emission end surface 32a by 10% will be described with reference to FIGS. 3A and 3B.

As the length L1 of one side of the glass rod 30 is made smaller than the diameter L2 of the emission end surface 32a, the light transmission efficiency of the glass rod 30 is reduced.

For example, in a case where the diameter L2 of the emission end surface 32a is 3.0 mm, the length L1 of one side of the glass rod 30 is 2.7 mm. In the case of this configuration, the emission end surface 32a protrudes from the incident end surface 30a of the glass rod 30 as shown in FIGS. 3A and 3B. For this reason, part of light emitted from the emission end surface 32a of the fiber 32 is not incident on the glass rod 30. In this case, the light transmission efficiency of the glass rod 30 is about 92%. Accordingly, in a case where the length L1 of one side of the glass rod 30 has a length shorter than the diameter L2 of the emission end surface 32a of the fiber 32 by 10% as a lower limit, a loss in the amount of light is in an allowable range.

Next, a case where the length L1 of one side of the glass rod 30 is larger than the diameter L2 of the emission end surface 32a by 10% will be described with reference to FIGS. 4A and 4B.

For example, in a case where the diameter L2 of the emission end surface 32a is 3.0 mm, the length L1 of one side of the glass rod 30 is 3.3 mm. In the case of this configuration, the incident end surface 30a of the glass rod 30 covers the entire emission end surface 32a of the fiber 32 as shown in FIGS. 4A and 4B. For this reason, the light transmission efficiency of the glass rod 30 is about 100%.

Here, generally, in order to reduce the unevenness of illumination emitted from the glass rod 30, it is preferable that the number of times of reflection of light in the glass rod 30 is 3 or more. In order to reflect light in the glass rod 30 three times, a length L3 of the glass rod 30 in the longitudinal direction needs to be 28.43 mm in a case where the length L1 of one side of the glass rod 30 is 3.3 mm.

Here, in order to reflect light in the glass rod 30 three times, the length L3 of the glass rod 30 needs to be about 25.84 mm in a case where the length L1 of one side of the glass rod is 3.0 mm.

That is, an increase in the length in a case where the length L1 of one side of the glass rod is 3.3 mm can be kept within 10% of the length in a case where the length L1 is 3.0 mm. Accordingly, it is possible to achieve a small size of the device while suppressing the unevenness of illumination.

From the above description, in a case where the length of one side of the glass rod 30 is increased, the length L3 of the glass rod 30 needs to be increased in order to ensure the number of times (three times) of reflection of light in the glass rod 30. However, in a case where the length L1 of one side of the glass rod 30 is larger than the diameter L2 of the emission end surface 32a by 10% as an upper limit, an increase in the length L3 of the glass rod 30 can be kept within 10%.

In the present embodiment, the incident end surface 30a of the glass rod 30 is adapted to cover the entire emission end surface 32a of the fiber 32 as shown in FIGS. 4A and 4B.

Next, the relationship between the size of the illumination light source 20 and an outer diameter of an incident end surface 22b of the second optical fiber bundle 22 will be described.

First, an etendue will be described.

In a case where an object is illuminated with light, which is emitted from a light source, via a lens, a trade-off relationship in which NA is increased in a case where a light spot of illumination light is to be reduced in size and a light spot is increased in size in a case where NA of illumination light is to be reduced is satisfied. In order to consider this trade-off relationship, the concept of "etendue" is introduced in the present invention.

An etendue is a physical quantity that is a product of a solid angle and an area. In a case where illumination light emitted from a light-emitting surface of the illumination light source 20 is condensed onto the second condensing optical system 21 and incident on a light-receiving surface (the incident end surface 22b of the second optical fiber bundle 22) as shown in FIG. 1, the area of the light-emitting surface of the illumination light source 20 is denoted by S 1, a solid angle from the light-emitting surface is denoted by $\Omega 1$, the area of the incident end surface 22b of the second optical fiber bundle 22 is denoted by S2, and a solid angle from the incident end surface 22b of the second optical fiber bundle 22 is denoted by $\Omega 2$, the following equation (1) is satisfied.

$$S1 \times \Omega1 = S2 \times \Omega2 \ ... \ (1)$$

Further, the following equation (2) needs to be satisfied as a condition in which light emitted from the illumination light source 20 is efficiently used without waste.

Etendue of light-emitting surface of illumination light source 20 <etendue of incident end surface 22b of second optical fiber bundle 22

Here, since the etendue of the illumination light source (LED) 20 is larger than the etendue of the excitation laser light source (LD) 10, it is desirable to increase an outer diameter of the fiber corresponding to the illumination light source 20, that is, the second optical fiber bundle 22. It is necessary to design the size of the light-emitting surface of the illumination light source 20 and the outer diameter of the incident end surface 22b of the second optical fiber bundle 22 such that light emitted from the illumination light source 20 is efficiently incident on the second optical fiber bundle 22.

First, assuming that the endoscope 100 is inserted into a human body, the diameter L2 of the fiber 32 is set to a maximum of 5 mm.

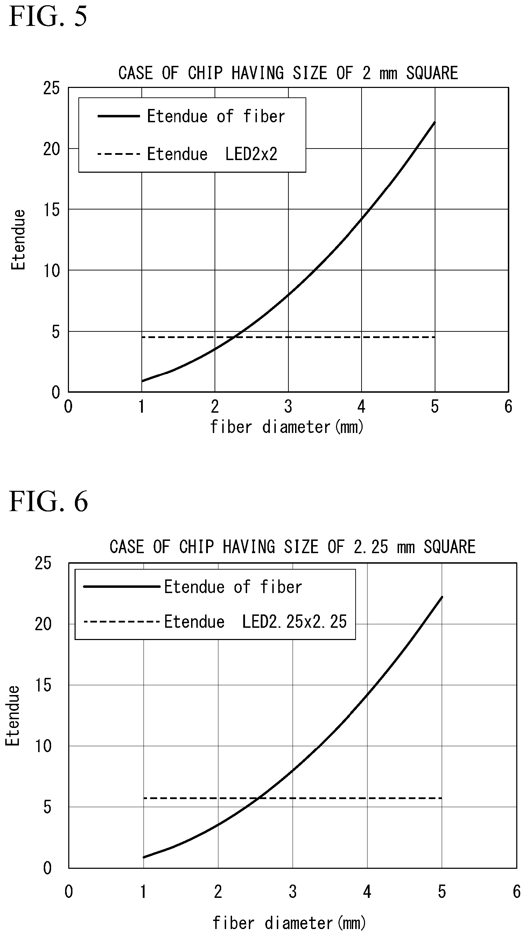

The relationship between the diameter of the second optical fiber bundle 22 in a case where the size of the illumination light source (LED) 20 is 2 mm × 2 mm and an etendue is shown in FIG. 5. A horizontal axis represents the diameter of the second optical fiber bundle 22, and a vertical axis represents an etendue.

Here, the relationship between the diameter of the second optical fiber bundle 22 in a case where the illumination light source 20 is in direct contact with the incident end surface 22b of the second optical fiber bundle 22 and an etendue is shown in FIG. 5 by a one-dot chain line. Since being determined depending on the size of the illumination light source 20 and NA of the second optical fiber bundle 22, an etendue has a constant value (etendue: 4.8) regardless of the diameter of the second optical fiber bundle 22.

In the present embodiment, the second optical fiber bundle 22 is a magnification optical system. Accordingly, as shown by a solid line in FIG. 5, the value of an etendue is also increased in a case where the diameter of the second optical fiber bundle 22 is increased.

The diameter of the second optical fiber bundle 22 is 2.4 mm at an intersection between the solid line and the one-dot chain line.

Accordingly, in a case where the size of the illumination light source (LED) 20 is 2 mm × 2 mm, it is preferable that the diameter of the second optical fiber bundle 22 is set to 2.4 mm or more and 5.0 mm or less.

Next, the relationship between the diameter of the second optical fiber bundle 22 in a case where the size of the illum7ination light source (LED) 20 is 2.25 mm × 2.25 mm and an etendue is shown in FIG. 6. A horizontal axis represents the diameter of the second optical fiber bundle 22, and a vertical axis represents an etendue.

In a case where the illumination light source 20 is in direct contact with the incident end surface 22b of the second optical fiber bundle 22, an etendue has a constant value (etendue: 6).

As shown in FIG. 6, the diameter of the second optical fiber bundle 22 at an intersection between the etendue (solid line) of the incident end surface 22b of the second optical fiber bundle 22 and an etendue (one-dot chain line) in a case where the illumination light source 20 is in direct contact with the incident end surface 22b of the second optical fiber bundle 22 is 2.6 mm.

Accordingly, in a case where the size of the illumination light source (LED) 20 is 2.25 mm × 2.25 mm, it is preferable that the diameter of the second optical fiber bundle 22 is set to 2.6 mm or more and 5.0 mm or less.

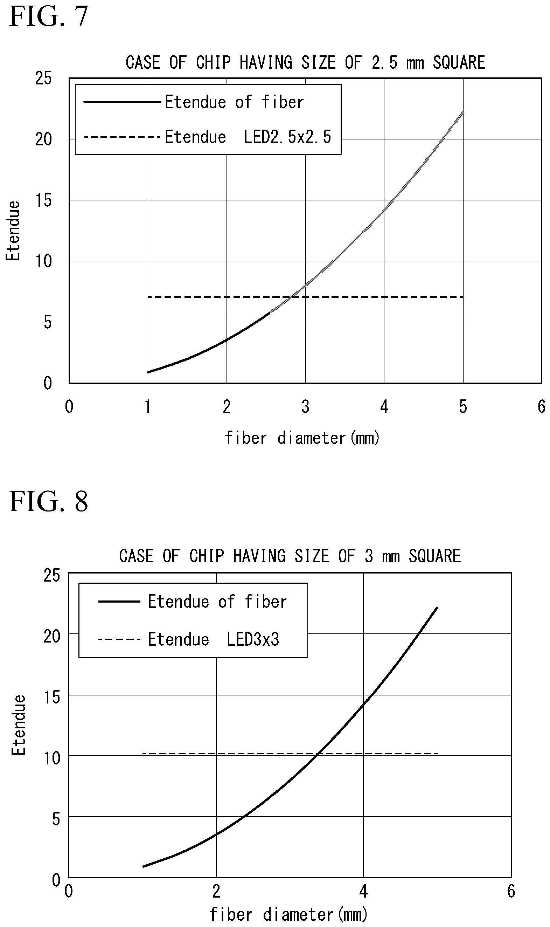

Next, the relationship between the diameter of the second optical fiber bundle 22 in a case where the size of the illumination light source (LED) 20 is 2.5 mm × 2.5 mm and an etendue is shown in FIG. 7. A horizontal axis represents the diameter of the second optical fiber bundle 22, and a vertical axis represents an etendue.

In a case where the illumination light source 20 is in direct contact with the incident end surface 22b of the second optical fiber bundle 22, an etendue has a constant value (etendue: 7).

As shown in FIG. 7, the diameter of the second optical fiber bundle 22 at an intersection between the etendue (solid line) of the incident end surface 22b of the second optical fiber bundle 22 and an etendue (one-dot chain line) in a case where the illumination light source 20 is in direct contact with the incident end surface 22b of the second optical fiber bundle 22 is 2.9 mm.

Accordingly, in a case where the size of the illumination light source (LED) 20 is 2.5 mm × 2.5 mm, it is preferable that the diameter of the second optical fiber bundle 22 is set to 2.9 mm or more and 5.0 mm or less.

Next, the relationship between the diameter of the second optical fiber bundle 22 in a case where the size of the illumination light source (LED) 20 is 3.0 mm × 3.0 mm and an etendue is shown in FIG. 8. A horizontal axis represents the diameter of the second optical fiber bundle 22, and a vertical axis represents an etendue.

In a case where the illumination light source 20 is in direct contact with the incident end surface 22b of the second optical fiber bundle 22, an etendue has a constant value (etendue: 10).

As shown in FIG. 8, the diameter of the second optical fiber bundle 22 at an intersection between the etendue (solid line) of the incident end surface 22b of the second optical fiber bundle 22 and an etendue (one-dot chain line) in a case where the illumination light source 20 is in direct contact with the incident end surface 22b of the second optical fiber bundle 22 is 3.4 mm.

Accordingly, in a case where the size of the illumination light source (LED) 20 is 3.0 mm × 3.0 mm, it is preferable that the diameter of the second optical fiber bundle 22 is set to 3.4 mm or more and 5.0 mm or less.

It is possible to improve a coupling effect from the second condensing optical system 21 to the second optical fiber bundle 22 by setting the diameter of the second optical fiber bundle 22 in consideration of an etendue as described above.

From the above description, in the present embodiment, light emitted from the illumination light source 20 is made to be incident on the second optical fiber bundle 22 by the second condensing optical system 21. Accordingly, since light emitted from the illumination light source 20 can be efficiently taken into the second optical fiber bundle 22, illumination light to be emitted from the glass rod 30 can be made uniform.

The shape of a cross-section of the glass rod 30 orthogonal to the longitudinal direction of the glass rod 30 has been a square shape, but may be a rectangular shape. In a case where the shape of the cross-section of the glass rod 30 is a rectangular shape, it is preferable that a difference between a long side and a short side is not large. In addition, the shape of the cross-section of the glass rod 30 orthogonal to the longitudinal direction of the glass rod 30 may be a polygonal shape (for example, a pentagonal shape, a hexagonal shape, or the like).

Further, the first condensing optical system 11 and the second condensing optical system 21 have been described as magnification optical systems, but may not be magnification optical systems.

[Modification example]

Next, a modification example of the present disclosure will be described, and the basic configuration of the modification example is the same as that of the above-mentioned embodiment. Accordingly, the same components as those of the above-mentioned embodiment will be denoted by the same reference numerals as the reference numerals of the above-mentioned embodiment and the description thereof will be omitted, and only differences between the modification example and the above-mentioned embodiment will be described.

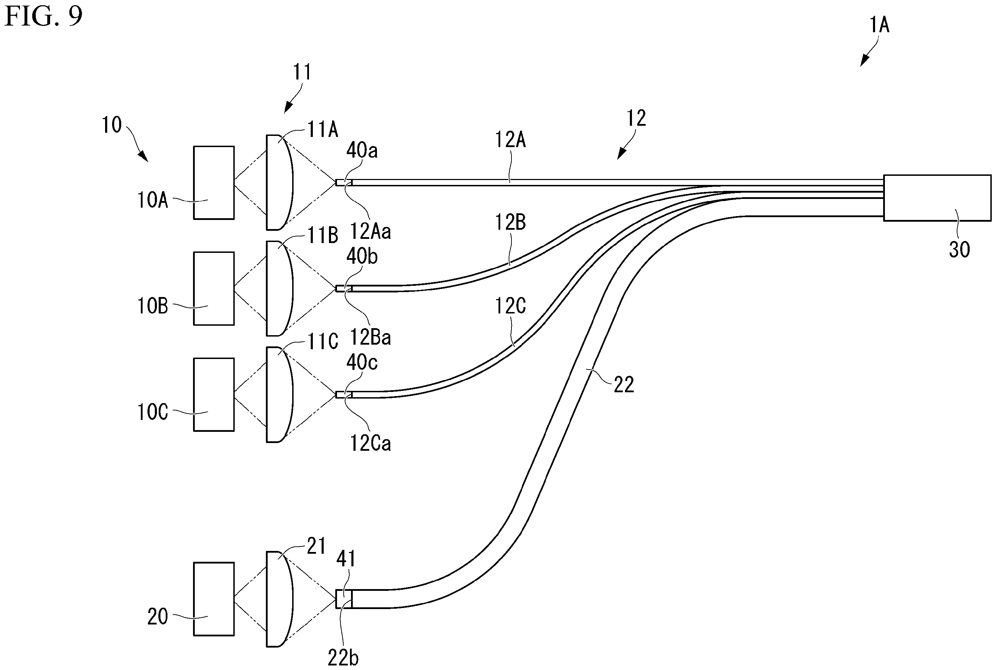

As shown in FIG. 9, in a light source device 1A for an endoscope of the present modification example, glass rods (second glass rods) 40a, 40b, and 40c are provided on incident end surfaces 12Aa, 12Ba, and 12Ca of a first optical fiber bundle 12, respectively, and a glass rod (second glass rod) 41 is provided on an incident end surface 22b of a second optical fiber bundle 22.

The glass rod 40a is provided in contact with the incident end surface 12Aa of a first fiber 12A. The glass rod 40b is provided in contact with the incident end surface 12Ba of a second fiber 12B. The glass rod 40c is provided in contact with the incident end surface 12Ca of a third fiber 12C.

Further, the glass rod 41 is provided in contact with the incident end surface 22b of the second optical fiber bundle 22.

The action of the glass rods 40a to 40c and 41 will be described.

Light emitted from an excitation laser light source 10 is condensed by a first condensing optical system 11, is incident on the glass rods 40a to 40c, and is guided through the glass rods 40a to 40c. Light, which is guided while being reflected in the glass rods 40a to 40c, is uniformized and is incident on the first optical fiber bundle 12.

Further, light emitted from the illumination light source 20 is condensed by a second condensing optical system 21, incident on the glass rod 41, and is guided through the glass rod 41. Light, which is guided while being reflected in the glass rod 41, is uniformized and is incident on the second optical fiber bundle 22.

Since the glass rods 40a to 40c and 41 are provided, the uniformized light is incident on the optical fiber bundles 12 and 22. As a result, loads on the optical fiber bundles 12 and 22 can be reduced.

The glass rods 40a to 40c have been provided on the incident end surfaces 12Aa to 12Ca of the fibers 12A to 12C, respectively, and the glass rod 41 has been provided on the incident end surface 22b of the second optical fiber bundle 22. However, the glass rods 40a to 40c may be provided at least only on the incident end surfaces 12Aa to 12Ca of the fibers 12A to 12C.

The embodiment and the modification example of the present disclosure have been described in detail above with reference to the drawings. In addition, the components shown in the embodiment and the modification example described above can be appropriately combined.

**Claims**

1. A light source device (1) for endoscope comprising:

   a plurality of excitation laser light sources (10);
   a first condensing optical system (11) that is formed of a plurality of optical systems condensing excitation light generated from the plurality of excitation laser light sources (10);
   a plurality of first optical fiber bundles (12) on which the excitation light condensed by the plurality of optical systems is incident;
   an illumination light source (20) that generates white light;
   a second condensing optical system (21) that condenses the white light emitted from the illumination light source (20);
   a second optical fiber bundle (22) on which the light emitted from the illumination light source (20) and condensed by the second condensing optical system (21) is incident; and
   a first glass rod (30) of which a first end surface (30a) is connected to emission end surfaces (12a, 22a) of the first and second optical fiber bundles (12, 22), first emission ends (12a) of the first optical fiber bundles (12) and a second emission end (22a) of the second optical fiber bundle (22) being bundled,
   wherein a connecting portion of an illumination optical system of an endoscope (100) is provided on a second end surface (30b) of the first glass rod (30) opposite to the first end surface (30a).

2. The light source device (1) for endoscope according to claim 1,

   wherein a shape of a cross-section of the first glass rod (30) orthogonal to a longitudinal direction of the first glass rod (30) is a square shape,
   the light emission ends of the first and second optical fiber bundles (12, 22) are bundled in a circular shape, and/or
   a length of one side of the cross-section of the first glass rod (30) orthogonal to the longitudinal direction is within a range larger or smaller than a diameter of the emission end surface of the emission end by 10%.

3. The light source device (1) for endoscope according to claim 1,

   wherein a diameter of a fiber bundle in which the emission ends of the first and second optical fiber bundles (12, 22) are bundled is 5.0 mm or less,
   a size of the illumination light source (20) is 2 mm square or more and 3 mm square or less, and/or
   a diameter of an incident end surface of the second optical fiber bundle (22) is 3.4 mm or more and 5.0 mm or less.

4. The light source device (1) for endoscope according to claim 1,

   wherein a diameter of a fiber bundle in which the emission ends of the first and second optical fiber bundles (12, 22) are bundled is 5.0 mm or less, and/or
   a diameter of an incident end surface of the second optical fiber bundle (22) is 2.4 mm or more and 5.0 mm or less in a case where a size of the illumination light source is 2 mm square.

5. The light source device (1) for endoscope according to claim 3,
   wherein a diameter of an incident end surface of the second optical fiber bundle (22) is 3.4 mm or more in a case where the size of the illumination light source (20) is 3 mm square.

6. The light source device (1) for endoscope according to any of the preceding claims, further comprising:

second glass rods (40a, 40b, 40c) that are disposed between the first condensing optical system (11) and the first optical fiber bundles (12),
wherein the excitation light condensed by the first condensing optical system (11) is incident on the second glass rods (40a, 40b, 40c).

7. The light source device (1) for endoscope according to any of the preceding claims, wherein the illumination light source (20) is an LED light source.

8. The light source device (1) for endoscope according to any of the preceding claims,
wherein at least the second condensing optical system (21) of the first condensing optical system (11) and the second condensing optical system (21) is a magnification optical system.

9. A system comprising the light source device (1) of any of the preceding claims and an endoscope (100).

# FIG. 1

LIGHT SOURCE DEVICE
FOR ENDOSCOPE

FIG. 2

EP 4 529 826 A1

# FIG. 3A

# FIG. 3B

# FIG. 4A

# FIG. 4B

FIG. 5

CASE OF CHIP HAVING SIZE OF 2 mm SQUARE

FIG. 6

CASE OF CHIP HAVING SIZE OF 2.25 mm SQUARE

FIG. 7

CASE OF CHIP HAVING SIZE OF 2.5 mm SQUARE

FIG. 8

CASE OF CHIP HAVING SIZE OF 3 mm SQUARE

FIG. 9

EP 4 529 826 A1

Europäisches
Patentamt

European
Patent Office

Office européen
des brevets

# EUROPEAN SEARCH REPORT

**Application Number**

EP 24 20 1441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2013 198547 A (FUJIFILM CORP) 3 October 2013 (2013-10-03) | 1,3-5, 7-9 | INV. A61B1/06 |
| Y | * paragraphs [0033], [0040], [0042], [0048], [0049] * * paragraphs [0050], [0072], [0074], [0076], [0125], [0135] * * figures 1,3,10, 12, 23 * | 2,6 | A61B1/07 |
| X | US 2013/345517 A1 (MORIMOTO YOSHINORI [JP] ET AL) 26 December 2013 (2013-12-26) | 1,3-5, 7-9 | |
| Y | * paragraphs [0064], [0071] - paragraph [0073] * * paragraph [0080] - paragraph [0083] * * paragraph [0106] - paragraph [0110] * * paragraphs [0127], [0133], [0134] * * paragraph [0137] - paragraphs [0143], [0193] * * figures 1,3,10,15,16 * | 2,6 | |
| Y | US 2022/095896 A1 (HANANO KAZUNARI [JP]) 31 March 2022 (2022-03-31) * paragraphs [0058], [0065], [0106], [0107] * * figures 19, 21 * | 2,6 | **TECHNICAL FIELDS SEARCHED** (IPC) A61B |

The present search report has been drawn up for all claims

| Place of search | Date of completion of the search | Examiner |
|---|---|---|
| Munich | 22 January 2025 | Doyle, Aidan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 1441

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2025

| Patent document cited in search report | Publication date | Patent family member(s) | Publication date |
|---|---|---|---|
| JP 2013198547 A | 03-10-2013 | JP 2013198547 A | 03-10-2013 |
| | | WO 2013140961 A1 | 26-09-2013 |
| US 2013345517 A1 | 26-12-2013 | CN 103505174 A | 15-01-2014 |
| | | JP 2014000301 A | 09-01-2014 |
| | | US 2013345517 A1 | 26-12-2013 |
| US 2022095896 A1 | 31-03-2022 | CN 113993438 A | 28-01-2022 |
| | | JP 7281544 B2 | 25-05-2023 |
| | | JP WO2020255398 A1 | 24-12-2020 |
| | | US 2022095896 A1 | 31-03-2022 |
| | | WO 2020255398 A1 | 24-12-2020 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016191888 A